**Europäisches Patentamt**

**European Patent Office**

**Office européen des brevets**

(11) Publication number: **0 059 032**
**A1**

(12) # EUROPEAN PATENT APPLICATION

(21) Application number: 82300522.8

(22) Date of filing: 02.02.82

(51) Int. Cl.³: **A 61 B 5/02**, G 01 N 33/48, **A 61 B 5/14**

(30) Priority: 03.02.81 US 231129

(43) Date of publication of application: 01.09.82
Bulletin 82/35

(84) Designated Contracting States: DE FR GB

(71) Applicant: Elings, Virgil B., 1155 Via Tranquila, Santa Barbara California 93110 (US)

(72) Inventor: Elings, Virgil B., 1155 Via Tranquila, Santa Barbara California 93110 (US)
Inventor: Briggs, Jonathan. 7090 Mary Mount Way, Goleta California 93017 (US)

(74) Representative: Smith, Philip Antony et al, REDDIE & GROSE 16 Theobalds Road, London WC1X 8PL (GB)

(54) Measurement of dye concentration in the bloodstream.

(57) To establish indicator dilution curves a fluorescent dye is injected into the bloodstream 12 and its concentration is measured by means of a catheter 10 including an optical fibre 14. Light from source 20 is passed through filter 24 into the outer end of the fibre 14 and emerges into the bloodstream at the remote end of the catheter. Here it stimulates fluorescent emission from the dye and light at the emitted wavelength (different from the exciting wavelength) travels back along the optical fibre, through a filter 28 to a detector 30.

# MEASUREMENT OF DYE CONCENTRATION IN THE BLOODSTREAM

The present invention relates to an apparatus and method for measuring indicator dilution curves in vivo in the bloodstream using a fluorescent dye.

In general, indicator dilution curves are created by injecting an indicator into a flowing fluid and having the indicator mix with the flowing fluid. At some point downstream from the original injection point, the concentration of the indicator in the fluid may then be sampled. The shape and magnitude of this dilution curve relative to time may be used to determine the amount of fluid flowing, volumes of chambers through which the fluid flows, time of recirculation of the indicator through the system, and other parameters involving the flowing fluid and the system through which it is flowing. In the present invention, the particular fluid under investigation is the blood flowing through the circulatory system of an animal and specifically a human.

In the past, several different types of indicators have been used to produce dilution curves. These indicators include colored dyes, heat (in particular, an injection of cold water), freon, radioactive atoms, etc.

One of the most common indicators used to produce a dilution curve has been indocyanine green dye. This green dye has a large absorption peak at approximately 800 nanometers which is in the infrared spectrum. Generally in the prior art, dye dilution curves are produced by injecting the green dye into the bloodstream and then measuring the dilution curve by actually withdrawing blood and looking at the concentration of green dye in the withdrawn blood. Specifically, the transmission of light through the withdrawn blood at one or more wavelengths may be measured using an optical densitometer. This particular technique has a major disadvantage in that blood must be withdrawn from the patient to obtain the dilution curve.

In the past there have been attempts made to obtain the green dye concentration and the resultant dilution curves using a fiber optic catheter located within the bloodstream. This prior technique has used a catheter with two or more sets of operating fibers and with infrared light of the proper frequency sent down one set of the fibers in the catheter. The set of fibers used to transmit the infrared light to the bloodstream is generally called the afferent fibers. The light which is sent down the afferent fibers is scattered off of blood cells in the bloodstream and reflected back into another different set of fibers in the catheter. The other set of fibers is generally called the efferent fibers. The scattered reflected light is of the same wavelength as the light sent down the afferent fibers. The green dye will act to attenuate the infrared light as the light passes out to the blood cells and back to the efferent fibers. The reflected light as attenuated by the green dye is therefore an inverse measurement of the green dye concentration.

It will be seen that the use of the above technique has a number of disadvantages. In particular, the in vivo green dye technique requires a catheter with at least two sets of fibers. Also, the amount of scattered light depends on the concentration and the orientation of the blood cells. The scattered light is thereby changed during the heartbeat since the flow of blood is not steady state but pulsates. The output signal produced from the light returned along the efferent fibers therefore includes a heartbeat artifact.

Another disadvantage with the in vivo green dye technique is that the blood is much more transparent in the infrared region than in other regions of light so that the scattering takes place from blood cells which are quite far from the end of the fibers. If the tip of the catheter including the sets of fibers is positioned close to the vessel wall, light may be scattered off the wall and back into the efferent fibers thereby providing an inaccurate measurement. Any change in the position of the catheter with respect to the vessel wall due to heartbeat or breathing will create an artifact on the signal. Catheters which have been made to provide for the in vivo green dye measurement therefore are normally constructed to include structures at the end of the catheter to keep the ends of the sets of fibers away from the vessel wall.

Another disadvantage with the green dye technique is that the transmission of light in the infrared region through the blood also varies with the oxygenation of the blood. The oxygenation in turn varies during the breathing cycle therefore giving rise to a breathing artifact in the output measurement.

- 3 -

In general, the concentration of the green dye in the blood is a complicated function of the amount of scattered light returned back through the efferent fibers and with the various artifacts. In addition, the geometry of the catheter is complicated.

Another indicator dilution curve techinque which has been used to some extent is the provision of heat as the indicator. In particular, cold water may be injection into the bloodstream and the temperature of the blood sampled by a thermistor positioned at the end of a catheter. There are, however, problems with the heat technique. Specifically, the thermistor, when mounted in the catheter, generally has a slow rise time such as a rise time of about one (1) second. Also, the thermistor is large compared to an optical fiber and therefore makes for a large catheter.

In some applications where one is looking at recirculation of the indicator through the body, such as when looking for a left to right shunt in the heart, it would be desirable to inject the indicator downstream from the thermistor, with the injection port and thermistor being on the same catheter. Unfortunately, as the cold water passes through the catheter and by the thermistor, heat will diffuse across the walls of the catheter and provide a large unwanted signal to the thermistor, making the detection of recirculation through the shunt difficult. Therefore, it would generally be necessary to provide for a separate catheter for the injection of the cold water so as not to mask the output signal from the thermistor.

## Summary of the Invention

The present invention provides for the use of a fluorescent dye or small fluorescent particles as the indicator for providing for in vivo measurements of dilution curves. A specific fluorescent dye which has been used is sodium fluoroscein but it is to be appreciated that other fluorescent dyes may be used or particles such as fluorescently dyed Dextran may be used. The words "fluorescent dye", are used to mean any molecule or particle that fluoresces. The fluorescent dye is injected into the bloodstream and the concentration of the dye is sampled in the bloodstream using a catheter which contains a single optical fiber or a bundle of fibers operating as a single fiber. The concentration of the fluorescent dye in the bloodstream is measured by sending light of one wavelength down the single fiber to excite the fluorescence in the dye, and then detecting the emitted fluorescent light, which is at a different wavelength, returning back along the same fiber. The intensity of the returning light is thereby a measurement of the concentration of the fluorescent dye in the bloodstream. For the particular example of sodium fluoroscein the exciting light would be in the blue region of the spectrum and the fluorescent light emitted by the dye is in the yellow-green region of the spectrum. Filters may be used so as to separate the light at the different wavelengths.

The fluorescent dye technique of the present invention has several advantages over the prior art techniques. It is not necessary to draw any blood as may have to be done with one green dye technique. In addition the catheter is

- 5 -

relatively simple in construction in that it contains only a single fiber or a plurality of fibers acting as a single fiber. In addition, the fluorescent dye technique of the present invention has a time response which is much faster than either the use of green dye or heat. For example, with the present invention the system may be made to have a rise time of less than one millisecond whereas the green dye technique when withdrawing blood has a rise time of about .6 to about .8 seconds. The use of heat provides for a rise time of about one (1) second.

The fluorescent dye technique of the present invention therefore provides for a faster response and for small children, where the circulation times are very fast, it is important to have a fast rise time so as to see the early appearance of the dilution curve.

Another advantage of the fluorescent dye techinque of the present invention is that the blue light is normally strongly attenuated by the blood. Blue light is attenuated about 100 times more strongly than the infrared light used in the green dye measurement. Because of this, the sampling volume for the fluorescent dye technique is very small and close to the fiber tip and, therefore, the system is not very susceptible to wall artifacts unless the end of the fiber is right against the wall. This would normally not be a problem since the end of the fiber may be recessed a small amount.

In addition, it has been determined that the signal produced by a measurement of the emitted fluorescent light does not depend on the oxygenation of the blood and therefore the measured signal would not normally include a breathing artifact. Also, since the fluorescent signal does not depend on light scattered from the blood cells the measured signal does not normally include a heartbeat artifact.

It has also been determined that the amount of fluorescent dye, such as sodium fluorescein, which must be injected so as to provide for the dilution curves can be relatively small. For example, dilution curves have been obtained using only 0.1 milligrams of sodium fluoroscein per injection. Typically the injection would be a larger quantity such as two (2) milligrams but the invention may use even larger quantities per injection. It should be noted that a standard fluorescent eye examination uses an injection of approximately five hundred (500) milligrams into the bloodstream. Therefore, the quantity of fluorescent used would not appear to create any health hazards.

## Brief Description of the Drawings

A clearer understanding of the invention will be had with reference to the following description and drawings wherein:

Figure 1 illustrates a general system of the present invention showing a catheter, including an optical fiber, located in the bloodstream;

- 7 -

Figure 2 illustrates in enlarged form the end of the fiber of Figure 1 in relation to the blood in the bloodstream;

Figure 3 is a graph showing the relationship of the amplitude of the output signal relative to the dye concentration;

Figure 4 is a first alternative structure for separating the light of the different wavelengths and including an optical fiber formed by a plurality of fibers;

Figure 5 is another alternative structure for separating the light of the different wavelengths

Figure 6 illustrates one use of the fluorescent dye technique of the present invention for measuring a heart shunt;

Figure 7 is a graph illustrating a normal dilution curve produced by the fluorescent dye technique of the present invention; and

Figure 8 is a graph illustrating a dilution curve wherein the patient has a heart shunt.

Description of the Preferred Embodiments

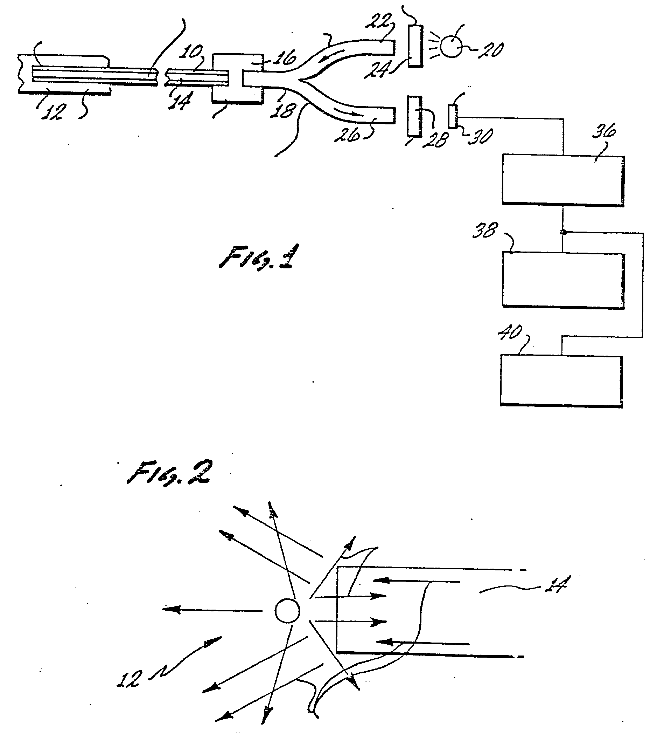

As can be seen in Figure 1, the apparatus and method of the present invention uses a catheter 10 located within the bloodstream such as an artery 12. The catheter 10 includes an optical fiber 14 which may either be a single fiber or a bundle of fibers acting as a single fiber.

The end of the catheter outside of the bloodstream is coupled through an optical coupler 16 to a device 18 for separating the incoming and outgoing light. In particular, the device 18 may be formed as an optical "Y" and such a device is commonly used in the optical field to provide for separating light energy.

In the particular example shown in Figure 1, a light source 20 provides light energy to one branch end 22 of the optical "Y" 18. The light from the light source 20 may be passed through a filter 24 so that the light entering the branch end 22 of the optical "Y" 18 has a relatively narrow range of wavelengths. As a specific example, the light passing into the branch end 22 may be blue light having wavelengths between 430 nanometers to 470 nanometers. This particular range of wavelengths is centered on a wavelength of 450 nanometers, called an isosbestic point, where the transmission of light through the blood does not depend on the oxygenation of the blood. The blue light, when exiting the optical fiber 14, is used to excite the fluorescent dye, in the fluid in the bloodstream, so that the filter 24 may be referred to as an exciter filter.

The light from the light source 20 is therefore filtered by the exciter filter 24 to produce exciter light which enters the branch end 22 of the optical "Y" 18. The exciter light is then coupled by the coupler 16 into the optical fiber 14 and is transmitted by the optical fiber down the length of the catheter 10. The exciter light exits the end of the fiber 14 to excite any fluorescent dye in the fluid in the bloodstream. Most of the exciter light actually exits the fiber 14 and excites the fluorescent dye to emit fluorescent light of a longer wavelength. This may be seen in greater detail with reference to Figure 2 wherein the emitted fluorescent light extends in all directions. As can be seen in Figure 2, a portion of the emitted light goes back into the optical fiber 14 to be transmitted back along the fiber 14.

If the exciter light is blue light ranging between 450 to 490 nanometers and if the fluorescent dye is sodium fluoroscein, the fluorescent light emitted by the dye is in the yellow-green region of the specturm. The fluorescent light is at longer wavelengths than the exciter light and, for example, may include wavelengths ranging between 510 to 600 nanometers. The returning fluorescent light is transmitted back up the fiber 14 and through the optical coupler 16 to the optical "Y" 18. A portion of the fluorescent light enters the branch end 22 and is lost for measurement purposes but another portion of the fluorescent light enters a second branch end 26 and is passed through a filter 28 to a photo-detector 30. The filter 28 may be referred to as a barrier filter and is designed to pass light only of the wavelengths representative of the fluorescent light such as the wavelengths ranging between 510 to 600 nanometers.

The filters 24 and 28 both exclude the passage of light except the light at the selected wavelengths, so that any of the exciter light which passes into the branch end 26 is not passed to the photodetector 30. For example, some of the exciter light may be reflected by the blood cells into the end of the fiber 14 within the bloodstream. This reflected light may be transmitted back along the fiber 14 to ultimately enter the branch end 26. However, the barrier filter 28 prevents the passage of the reflected exciter light to the photodetector 30. The photodetector 30 therefore sees only light representative of the concentration of the fluorescent dye. The output signal from the photodetector taken over a period of time forms a dilution curve representative of the concentration of the fluorescent dye relative to time.

The end of the fiber 14 within the bloodstream is primarily sensitive to fluorescent light produced by the dye which is close to the end of the fiber. Therefore, the fluorescent light transmitted back along the fiber 14 is representative primarily of the dye close to the end of the fiber. This is because the exciting light such as the blue light is strongly attenuated by the red blood and the blue light is at its strongest level only near the end of the fiber. In addition, since the fluorescent dye emits the fluorescent light, such as the yellow-green light, in all directions, the dye near the end of the fiber emits the largest fraction of the light into the fiber 14. Therefore, the dye which is further away from the end of the fiber 14 within the bloodstream has a much lower effect on the value of the fluorescent light transmitted back through the fiber 14.

If the optical "Y" 18 is symmetrical, then half of the returning light, which includes the fluorescent light and any of the reflected exciter light, is passed to the branch end 22 and half of the returning light is passed to the branch end 26. However, as indicated above, any returning exciter light cannot pass through the barrier filter 28 and therefore the optical detector such as the photodetector 30 provides for an output signal representative of the concentration of the fluorescent dye in the blood. The output signal from the detector 30 relative to the concentration may be seen with reference to Figure 3 which is a graph plotting this relationship.

It has been determined that at low to moderate concentrations of fluorescent dye, such as concentrations of sodium fluoroscein below ten (10) milligrams per liter, the measured fluorescent light is directly proportional to the concentration of fluorescent dye in the blood. This is shown by the portion 32 of the graph of Figure 3. At higher concentrations the exciter light, such as the blue light, is attenuated by the blood and by the fluorescent dye and therefore the relative volume of blood and dye observed at the end of the fiber becomes relatively less. Therefore the output signal does not increase as fast as the increase in concentration of dye. This is shown by the portion 34 of the graph of Figure 3. Therefore, if it is desirable to provide for a direct relationship in the measurements, the measurements may be made using relatively low concentrations of dye.

Returning to Figure 1, the output from the photo-detector may be amplified by an amplifier 36 and coupled to various output devices, such as a chart recorder 38 to produce a visual indication of the dilution curve, and a computer 40 to provide for various uses of the dilution curve as part of further calculations.

As an example, one use for the fluorescent dye indicator dilution curve is to provide the intravascular indicator curve used in the double indicator dilution method of measuring lung water. In the double indicator method of measuring lung water, two indicators are injected simultaneously and detected with a single catheter in an artery. The fluorescent dye technique of the present invention may be used in combination with another indicator such as heat and then detected with a single catheter in the artery. The single catheter would include a thermistor to measure the thermodilution curve and the optical fiber 14 to measure the fluorescent dye dilution curve. This technique would allow for the measurement of lung water without withdrawing blood.

0059032

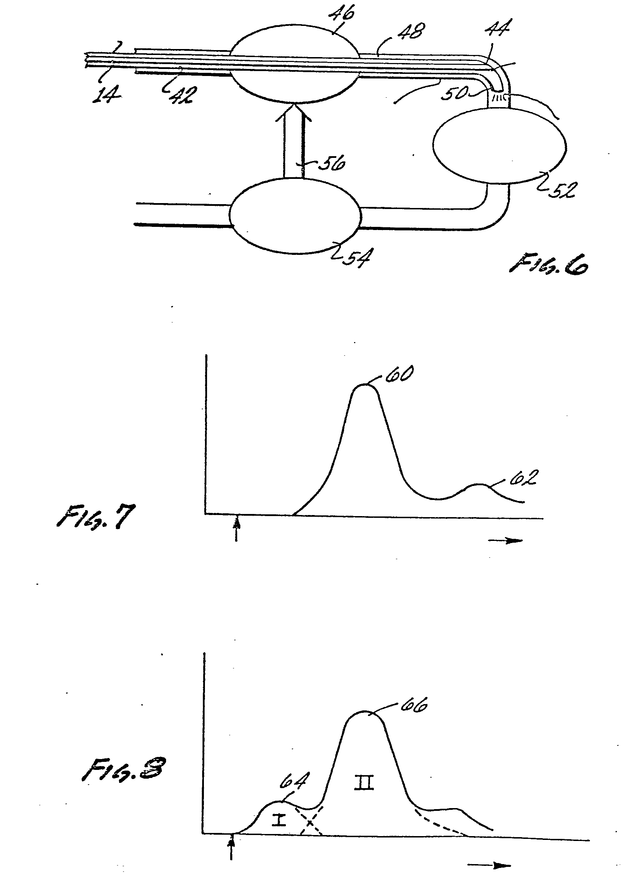

Another use of the dilution curve produced by the fluorescent dye technique of the present invention would be for the detection of shunts in the heart. This may be seen for example with reference to Figure 6 wherein a detection of a left to right shunt is provided. In Figure 6, a catheter 42 including the fiber 14 terminating at a position 44 along the catheter is inserted through the right heart 46 into the pulmonary artery 48. The catheter 42 may also include a tube so as to provide for the injection of the fluorescent dye into the pulmonary artery through an injection port 50 at the end of the catheter. The dye passes through the lungs 52 and to the left heart 54.

If there is a shunt as shown by the shunt 56 then some of the dye is shunted directly to the right heart and makes an early appearance at the end position 44 of the fiber 14. Specifically, the dilution curve will be modified in accordance with the early appearance of the fluorescent dye at the end position 44 of the fiber 14. This may be seen with reference to Figures 7 and 8 wherein Figure 7 is a normal dilution curve and Figure 8 is a dilution curve when there is a shunt 56 between the left and right heart.

In the normal dilution curve shown in Figure 7 the amplitude of the signal representative of the fluorescent light is shown relative to time and as can be seen the signal starts to rise at some point after the time of injection of the fluorescent dye. The signal rises to a maximum point 60 representing the first circulation of the dye into the arteries and then back through the veins before the dye then returns

- 14 -

again to the pulmonary artery. Subsequent recirculation of the dye provides for a second lower point 62 and ultimately the dye is evenly distributed throught the bloodstream. If there is a shunt in the heart, the dilution curve will have a different appearance as shown in Figure 8.

As can be seen in Figure 8, after the injection of the dye, the dilution curve rises to a small first peak point 64 which occurs early and is due to the shunt in the heart 56. A second larger maximum point 66 represents the first normal circulation. It can be seen therefore that the shunt in the heart may be recognized by the shape of the curve and it is not necessary when making such a determination to provide for any calibration of the dilution curve. However, it is possible to provide for a proportional representation of the circulation through the shunt as opposed to the normal circulation by comparing the areas designated I and II in Figure 8. This gives a proportional ratio of the flow through the shunt relative to the flow into the arteries.

Another use of the apparatus is in the measurement of the ejection fraction of either the left or right ventricle of the heart. This measurement has been performed using thermodilution and green dye dilution curves as described in the book Dye Curves - The Theory and Practice of Indicator Dilution, Edited by D.A. Bloomfield, University Park Press, Baltimore, 1974. With the present apparatus, the measurement would consist of injecting the

fluorescent dye in our just upstream of the ventricle and measuring the concentration of the dye just downstream from the ventricle. As the ventricle beats, the dilution curve will consist of steps of successively lower concentrations of dye, the ratio of concentrations on successive steps being $\frac{C}{C'} = 1-E$ where C is the concentration of dye ejected during one beat, and C' is the concentration of dye ejected during the previous beat, and E is the ejection fraction of the ventricle which is the fraction of the volume of the ventricle which is ejected during each heart beat. This measurement of ejection fraction depends only on the shape of the dilution curve and therefore the calibration of the system does not need to be known, i.e., the ratio of the dye concentration on successive beats does not depend on the overall sensitivity of the system.

For the measurement of ejection fraction, the measurement system must have a fast time response in order to define the steps in the dilution curve. A time response of less than .030 seconds is preferred, and this is not psssible with thermistors mounted on catheters or with green dye systems which pull blood to measure the dilution curve. The fluorescent dye dilution system can have a time response which is much less than .030 seconds.

Another application of the present invention is in the measurement of the flow through the heart called cardiac output. For this measurement one would inject the fluorescent dye upstream from or in the right heart and sample the dilution curve in the pulmonary artery. The cardiac output is calculated as the amount of dye injected (in mg.) divided by the area under the dilution curve $\int_0^\infty C(t)dt$ where $C(t)$ is the concentration (in mg./liter) of indicator as a function of time. In this application, the calibration of the system must be known. Cardiac output is usually measured using a thermal indicator, typically in the form of a 10cc of an ice-cold 5% dextrose solution, and a thermistor for the detector of the concentration of the indicator. If one wishes to measure the cardiac output of a patient on an almost continuous basis, say every 5 minutes, then the thermodilution technique cannot be used because the patient would receive too much fluid. Here the present invention would have an advantage because the amount of dye which needs to be injected to form a dilution curve is very small, as small as 0.1 mg. For one injection every 5 minutes, the patient would receive about 30 mg. of dye in one day, compared to 500 mg. which is injected during a standard fluorescent eye exam. If a thermistor were mounted on the same catheter with the optical fiber, one could calibrate the fluorescent dye measurement system periodically with thermodilution, i.e., one would set the calibration of the system such that the cardiac output calculated from the fluorescent dilution curve would equal that measured by the thermodilution. This calibration could be performed daily, for example, and between calibrations the cardiac output would be measured only from the fluorescent dilution curve.

In all these applications the fluorescence dilution curve is measured from a baseline signal which need not be zero. For instance, even though the patient had a background level of fluorescent dye already in the blood from previous measurements, a new fluorescence dilution curve can be measured on top of this background.

Although the first embodiment of the invention has been described with reference to the use of an optical "Y" to separate the exciter light and the fluorescent light, it is to be appreciated that other structures may be used. For example, Fig. 4 illustrates the use of a notched device 70 which provides for the light being separated into the different wavelengths. In Fig. 4 the device 70 is coupled to the fiber 14 and the device 70 includes a notch 72. Also, as shown in Fig. 4, the optical fiber 14 may be composed of a plurality of fibers operating as a single fiber. The exciter light, such as the blue light, is coupled to the device 70 through the notch 72. The returning fluorescent light passes back along the device 70 and passes through the barrier filter 28 to the detector 30. Again, a portion of the fluorescent light is lost at the notch 72 but a sufficient amount of the fluorescent light passes through the detector 30.

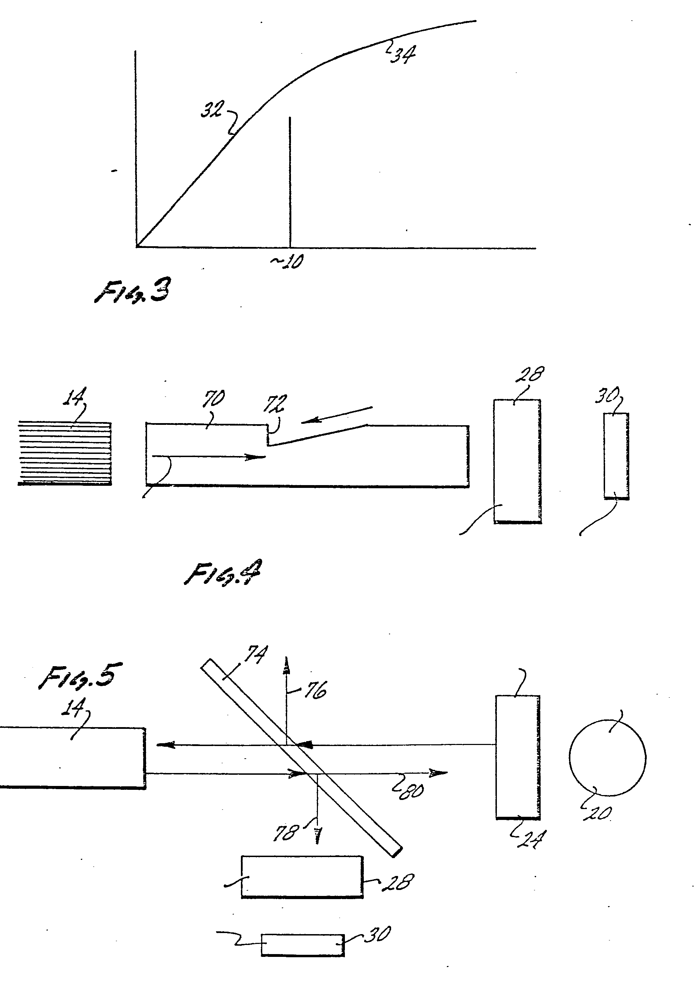

Another alternative structure for providing for the separation of the light at the different wavelengths is shown in Fig. 5. In Fig. 5 the light from the light source 20 passes through the exciter filter 24 to provide for the exciter light, such as the blue light. This exciter light passes through a half silvered mirror 74 to the optical fiber 14. A portion of the exciter light is lost as shown by arrow 76. The returning fluorescent light, such as the yellow-green light, returns along the fiber 14 and strikes the half-silvered mirror 74 to be reflected downward, as shown by arrow 78, and then on to the detector 30 through the barrier filter 28. A portion of the fluorescent light is lost as shown by arrow 80.

It is also to be appreciated that the photodetector 30 may be formed from any appropriate structure such as a photomultiplier tube or a solid state photodetector. The light source 20 may be an incandescent bulb or other types of light sources such as a flash lamp or a laser.

It is also to be appreciated that different fluorescent dyes may be used and that these dyes may fluoresce at different wavelengths than those described in the present invention. However, a preferred dye is sodium fluoroscein since this dye is excited by blue light and the blue light has the advantage of being attenuated strongly in the blood. This in turn makes the measurement occur very close to the end of the fiber and the measurement therefore is not effected by the different artifacts as described above.

Although the invention has been described with reference to particular embodiments, it is to be appreciated that various adaptations and modifications may be made and the invention is only to be limited by the appended claims.

CLAIMS

1. Apparatus for providing for the measurement of the concentration of an injected fluorescent dye in the fluid in the bloodstream and with the fluorescent dye providing for the emitting of fluorescent light at first particular wavelengths when excited by light at second particular wavelengths, including

a catheter for insertion into the bloodstream and with the catheter including an optical fiber extending substantially along the length of the catheter from a first end and at a first position within the bloodstream to a second end at a second position without the bloodstream and with the first end of the optical fiber adjacent the fluid within the bloodstream to transmit light along the optical fiber to and from the fluid within the bloodstream from and to the second end of the optical fiber,

a source of light coupled to the second end of the optical fiber for producing light at the second particular wavelengths for transmission along the length of the optical fiber for exiting the first end of the optical fiber to excite the fluorescent dye in the fluid in the bloodstream adjacent the first end to emit fluorescent light at the first particular wavelengths and with a portion of the fluorescent light at the first particular wavelengths entering the first end of the optical fiber for transmission along the length of the optical fiber to the second end, and

means coupled to the second end of the optical fiber for detecting the fluorescent light at the first particular

0059032

## Claim 1 - continued

wavelengths transmitted along the optical fiber and for producing output signals representative of the concentration of the fluorescent dye in the fluid in the bloodstream.

2. The apparatus of claim 1 additionally including means coupled to the second end of the optical fiber and to the source of light and to the means for detecting for separating the light at the first and second particular wavelengths.

3. The apparatus of claim 2 wherein the means for separating includes an optical "Y" and with the source of light coupled to one branch of the "Y" and the means for detecting coupled to the other branch of the "Y".

4. The apparatus of claim 2 wherein the means for separating includes a half silvered mirror.

5. The apparatus of claim 2 wherein the means for separating includes an optical fiber having a notched portion and with the source of light coupled to the notched portion.

6. The apparatus of claim 2 wherein the means for separating includes at least one filter for passing only the fluorescent light at the first particular wavelengths to the means for detecting.

0059032

7. The apparatus of claim 1 wherein the optical fiber is formed by a single fiber.

8. The apparatus of claim 1 wherein optical fiber is formed by a plurality of fibers operating as a single optical fiber.

9. The apparatus of claim 1 wherein the fluorescent dye is sodium fluoroscein and the source of light produces blue light and the means for detecting is responsive to yellow-green light.

10. The apparatus of claim 9 wherein the source of light produces blue light ranging between 430 to 470 nanometers and the means for detecting is responsive to yellow-green light ranging between 510 to 600 nanometers.

11. A method of measuring the concentration of an injected fluorescent dye in the fluid in the bloodstream wherein the fluorescent dye provides for the emitting of fluorescent light at first particular wavelengths when excited by light at second particular wavelengths, including the following steps:

providing a catheter including an optical fiber extending substantially along the length of the catheter from a first end to a second end,

- 22 -

Claim 11 - continued

inserting the catheter into the bloodstream to have the first end within the bloodstream and the second end without the bloodstream and with the first end of the optical fiber adjacent the fluid within the bloodstream to transmit light along the optical fiber to and from the fluid within the bloodstream from and to the second end of the optical fiber,

producing light at the second particular wavelengths and transmitting the produced light along the length of the optical fiber to exit the first end of the optical fiber to excite the fluorescent dye in the fluid in the bloodstream adjacent the first end to emit fluorescent light at the first particular wavelengths and with a portion of the fluorescent light at the first particular wavelengths entering the first end of the optical fiber transmitted along the length of the optical fiber to the second end, and.

detecting the fluorescent light at the first particular wavelengths transmitted along the optical fiber and producing output signals representative of the concentration of the fluorescent dye in the fluid in the bloodstream.

12. The method of claim 11 additionally including the step of separating the light at the first and second particular wavelengths before detecting the fluorescent light.

13. The method of claim 12 wherein the step of separating includes the use of an optical "Y".

14. The method of claim 12 wherein the step of separating includes the use of a half silvered mirror.

15. The method of claim 12 wherein the step of separating includes the use of an optical fiber having a notched portion.

16. The method of claim 12 wherein the step of separating includes at least one filter for passing only the fluorescent light at the first particular wavelengths.

17. The method of claim 11 wherein the optical fiber is formed by a single fiber.

18. The method of claim 11 wherein the optical fiber is formed by a plurality of fibers operating as a single optical fiber.

19. The method of claim 11 wherein the fluorescent dye is sodium fluoroscein and the produced light is blue light and the detected light is yellow-green light.

20. The method of claim 19 wherein the produced light ranges between 430 to 470 nanometers and the detected light ranging between 510 to 600 nanometers.

21. Apparatus for providing for the measurement of the concentration of an injected fluorescent dye in the fluid in the bloodstream and with the fluorescent dye providing for the emitting of fluorescent light at first particular wavelengths when excited by light at second particular wavelengths and with a source of light for producing light at the second particular wavelengths and a means for detecting the fluorescent light at the first particular wavelengths, including,

a catheter for insertion into the bloodstream and with the catheter including an optical fiber extending substantially along the length of the catheter from a first end and at a first position within the bloodstream to a second end at a second position without the bloodstream and with the first end of the optical fiber adjacent the fluid within the bloodstream to transmit light along the optical fiber to and from the fluid within the bloodstream from and to the second end of the optical fiber,

the produced light at the second particular wavelengths transmitted along the length of the optical fiber for exiting the first end of the optical fiber to excite the fluorescent dye in the fluid in the bloodstream adjacent the first end to emit fluorescent light at the first particular wavelengths and with a portion of the fluorescent light at the first particular wavelengths entering the first end of the optical fiber and transmitted along the length of the optical fiber to the second end, and

Claim 21 - continued

      the fluorescent light at the first particular wavelengths transmitted along the optical fiber representative of the concentration of the fluorescent dye in the fluid in the bloodstream.

      22.  The apparatus of claim 21 wherein the optical fiber is formed by a single fiber.

      23.  The apparatus of claim 21 wherein the optical fiber is formed by a plurality of fibers operating as a single optical fiber.

F16.1

F16.2

Fig.3

Fig.4

Fig.5

FIG. 6

FIG. 7

FIG. 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | FR-A-2 302 526 (MAX PLANCK GESELLSCHAFT) *Page 1, line 1 - line 20; page 7, line 4 - line 41; page 10, line 1 - page 11, line 10; figures 1,4,5* | 1,2,11 ,12,21 | A 61 B 5/02 G 01 N 33/48 A 61 B 5/14 |
| A | EP-A-0 003 015 (H.M.SHAPIRO) *Abstract; page 1, line 15 - line 24; page 3, line 16 - page 5, line 32; figures 1,2* | 1-3,6, 8-13, 16-18, 20,21, 23 | |
| A | CH-A- 617 769 (APPLIED PHOTOPHYSICS LIMITED) *Abstract; page 3, right-hand column, line 57 - page 4, right-hand column, line 37; page 5, right-hand column, line 19 - line 58; figures 1,2,5-7* | 1-3,11 -13,21 | **TECHNICAL FIELDS SEARCHED (Int. Cl. ³)** |
| A | ANALYTICAL CHEMISTRY, vol. 49, no. 13, November 1977, pages 2051-2053, Columbus Ohio (USA) R.M.SMITH et al.: "Design and evaluation of a fiber optic fluorometric flow cell" *The whole document* | 1-3,6- 8,11- 13,16- 18,21- 23 | A 61 B 5/02 A 61 B 5/14 G 01 N 33/48 G 01 N 21/64 |

The present search report has been drawn up for all claims

| Place of search THE HAGUE | Date of completion of the search 28-04-1982 | Examiner ZILLIOX J.M. |
|---|---|---|

| | DOCUMENTS CONSIDERED TO BE RELEVANT | | Page 2 |
|---|---|---|---|
| Category | Citation of document with indication, where appropriate, of relevant passages | Relevant to claim | CLASSIFICATION OF THE APPLICATION (Int. Cl. 3) |
| A | DE-A-2 264 433 (SIEMENS AG)<br><br>*Page 1, line 1 - page 2, line 24; page 3, line 7 - page 4, line 23; figure 1* | 1,2,6-8,11, 12,16-18,21-23 | |
| A | FR-A-2 263 510 (INTERNATIONAL DIAGNOSTIC TECHNOLOGY, INC.)<br><br>*Page 2, line 24 - page 3, line 27; page 9, line 26 - page 10, line 1; figures 8-10* | 1-3,6-9,11, 12,13, 16-19, 21-23 | |
| A | CLINICAL CHEMISTRY, vol. 19, no. 8, 1973, pages 871-882, Winston/Salem (USA)<br>T.O.TIFFANY et al.: "Fluorometric fast analyzer: some applications to fluorescence measurements in clinical chemistry". *Pages 871-874* | 9,10, 19,20 | TECHNICAL FIELDS SEARCHED (Int. Cl. 3) |
| A | FR-A-2 394 788 (MAX PLANCK GESELLSCHAFT)<br>*Page 1, line 31 - page 2, line 1; page 4, line 40 - page 6, line 17; figure 1* | 1,7,17 | |

The present search report has been drawn up for all claims

| Place of search | Date of completion of the search | Examiner |
|---|---|---|
| THE HAGUE | 28-04-1982 | ZILLIOX J.M. |

European Patent Office

| DOCUMENTS CONSIDERED TO BE RELEVANT | | | Page 3 |
|---|---|---|---|
| Category | Citation of document with indication, where appropriate, of relevant passages | Relevant to claim | CLASSIFICATION OF THE APPLICATION (Int. Cl. 3) |
| A | US-A-4 109 647 (M.D.STERN U.S.A. SECRETARY OF THE DEPARTMENT OF HEALTH EDUCATION AND WELFARE) *Abstract, column 5, line 22 - line 66; figure 1* | 4,14 | |
| | | | TECHNICAL FIELDS SEARCHED (Int. Cl. 3) |

The present search report has been drawn up for all claims

| Place of search | Date of completion of the search | Examiner |
|---|---|---|
| THE HAGUE | 28-04-1982 | ZILLIOX J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03 82